# EUROPEAN PATENT SPECIFICATION

(11) **EP 1 301 526 B1**
(45) Date of publication and mention of the grant of the patent: **18.06.2008**
(21) Application number: 01949791.6
(22) Date of filing: 20.07.2001
(51) Int. Cl.: C07J 5/00, C07J 31/00, C07J 3/00

(54) **OXIDATION PROCESS FOR PREPARING THE INTERMEDIATE 6.ALPHA.,9.ALPHA.-DIFLUORO-11.BETA.,17.ALPHA.-DIHYDROXY-16.ALPHA.-METHYL-ANDROST-1,4-DIEN-3-ONE 17.BETA.-CARBOXYLIC ACID**
OXIDATIONSVERFAHREN ZUR HERSTELLUNG DES ZWISCHENPRODUKTES 6.ALPHA.,9.ALPHA.-DIFLUORO-11.BETA.,17.ALPHA.-DIHYDROXY-16.ALPHA.-METHYL-ANDROST-1,4-DIEN-3-ONE 17.BETA.-KARBONSÄURE
PROCEDE D'OXYDATION POUR LA PREPARATION DE L'INTERMEDIAIRE ACIDE 6 ALPHA, 9 ALPHA-DIFLUORO-11 BETA,17 ALPHA-DIHYDROXY-16.ALPHA-METHYL-ANDROST-1,4-DIEN-3-ONE 17 BETA-CARBOXYLIQUE

(30) Priority: 21.07.2000 GB 0017988
(43) Date of publication of application: 16.04.2003
(73) Proprietor: GLAXO GROUP LIMITED, Greenford, Middlesex UB6 ONN (GB)
(72) Inventor: ALBINSON, Frederick David, GlaxoSmithKline, Stevenage, Hertfordshire SG1 2NY (GB); COOTE, Steven John, GlaxoSmithKline, Stevenage, Hertfordshire SG1 2NY (GB); ROBINSON, John Malcolm, GlaxoSmithKline, Stevenage, Hertfordshire SG1 2NY (GB)
(74) Representative: Walker, Ralph Francis
(86) International application number: PCT/GB2001/003289
(87) International publication number: WO 2002/008243

(56) References cited:
- WO-A-01/62722
- WO-A-95/18621
- WO-A-97/24365
- DE-A- 1 964 356
- GB-A- 2 018 256
- US-A- 4 198 336
- PHILLIPPS G H ET AL: "SYNTHESIS AND STRUCTURE-ACTIVITY RELATIONSHIPS IN A SERIES OF ANTIINFLAMMATORY CORTICOSTEROID ANALOGUES, HALOMETHYL ANDROSTANE- 17BETA-CARBOTHIOATES AND-17BETA-CARBOSELENOATES" JOURNAL OF MEDICINAL CHEMISTRY, AMERICAN CHEMICAL SOCIETY. WASHINGTON, US, vol. 37, no. 22, 1 October 1994 (1994-10-01), pages 3717-3729, XP002025925 ISSN: 0022-2623
- KERTESZ, DENIS J. ET AL: "Thiol esters from steroid 17.beta.-carboxylic acids: carboxylate activation and internal participation by 17.alpha.-acylates" J. ORG. CHEM. (1986), 51(12), 2315-28 , XP002181480

## Description

The present invention relates to a novel process for the synthesis of a known intermediate, useful in the preparation of anti-inflammatory steroids. There is also provided a new physical form of the intermediate which has improved handling properties

6α, 9α-difluoro-11β-hydroxy-16α-methyl-17α-propionyloxy-3-oxoandrosta-1, 4-diene-17β-carbothioic acid, S-fluoromethyl ester (Formula A) was first described as an anti-inflammatory steroid by US Patent No. 4,335,121. This compound is also known by the generic name of fluticasone propionate and has since become widely known as a highly effective steroid in the treatment of inflammatory diseases, such as asthma and chronic obstructive pulmonary disease (COPD).

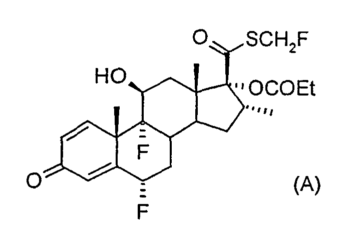

Additionally, 6α, 9α-difluoro-11β-hydroxy-16α-methyl-3-oxo-17α-propionyloxy-androsta-1,4-diene-17β-carbothioic acid S-(2-oxo-tetrahydro-furan-3-yl) ester (Formula B) was described in WO 97/24365 as a class of hydrolysable steroids with lactone derivatives. This compound possesses useful anti-inflammatory activity whilst having little or no systemic activity.

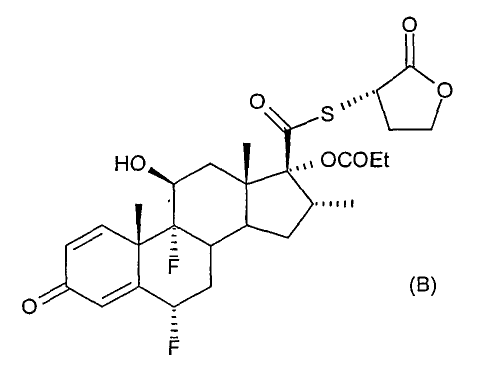

Currently there is considerable interest in compounds of formula (A) and (B) as anti-inflammatory and anti-allergic compounds in the treatment of asthma and other inflammatory diseases.

US 4,335,121 and WO 97/24365 describe preparations of fluticasone propionate and 6α, 9α-difluoro-11β-hydroxy-16α-methyl-3-oxo-17α-propionyloxy-androsta-1,4-diene-17β-carbothioic acid S-(2-oxo-tetrahydro-furan-3-yl) ester, respectively which utilise a common starting material, namely 6α, 9α-difluoro-11β-hydroxy-16α-methyl-3-oxo-17α-hydroxy-androsta-1,4-diene-17β-carboxylic acid (Formula I). However, this hydroxy acid androstane derivative compound is an extremely costly starting material for preparing quantities of steroids of formulae (A) and (B). See J. Org. Chem. vol 51(12), 2315-28 (1986) and DE-A-1,964,356 for the preparation of compound (I).

Thus, according to the present invention we provide a novel process for the preparation of a compound of formula (I) which comprises the following step: 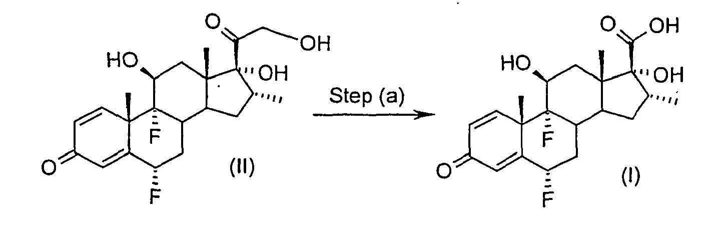 wherein step (a) comprises oxidation of a solution containing the compound of formula (II).

Preferably, step (a) will be performed in the presence of a solvent comprising methanol, water, tetrahydrofuran, dioxan or diethylene glygol dimethylether. For example, so as to enhance yield and throughput, preferred solvents are methanol, water or tetrahydrofuran, and more preferably are water or tetrahydrofuran, especially water and tetrahydrofuran as solvent. Dioxan and diethylene glygol dimethylether are also preferred solvents which may optionally (and preferably) be employed together with water.

Preferably, the solvent will be present in an amount of between 3 and 10vol relative to the amount of the starting material (1wt.), more preferably between 4 and 6 vol., especially 5 vol.

If desired, the solution containing the compound of formula (II) may be cooled prior to oxidation eg. to a temperature less than approximately 10°C.

Preferably the oxidising agent is present in an amount of 1-9 molar equivalents relative to the amount of the starting material.

The oxidation step utilises air and/or oxygen. The solvent used in said reaction will preferably be methanol.

Preferably, step (a) will involve incubating the reagents at room temperature or a little warmer, say around 25 °C eg for 2 hours.

The compound of formula (I) is then isolated by crystallisation from the reaction mixture by addition of an anti-solvent. A suitable anti-solvent for compound of formula (I) is water. Surprisingly we have discovered that it is highly desirable to control the conditions under which the compound of formula (I) is precipitated by addition of anti-solvent eg water. When the crystallisation is performed using chilled water (eg water/ice mixture at a temperature of 0-5 °C) although better anti-solvent properties may be expected we have found that the crystalline product produced is very voluminous, resembles a soft gel and is very difficult to filter. Without being limited by theory we believe that this low density product contains a large amount of solvated solvent within the crystal lattice By contrast when conditions of 10 °C or higher are used (eg around ambient temperature) a granular product of a sand like consistency which is very easily filtered is produced. Under these conditions, crystallisation typically commences after around 1 hour and is typically completed within a few hours (eg 2 hours). Without being limited by theory we believe that this granular product contains little or no of solvated solvent within the crystal lattice.

Consequently, according to the invention we provide a process for preparing a compound of formula (I) which comprises the steps of
(a) oxidation of a solution containing the compound of formula (II) (see above) followed by
(b) precipitation of the compound of formula (I) in crystalline form from the reaction mixture by addition of an anti-solvent under temperature conditions of 10 °C or higher.

Preferably the anti-solvent is water. Preferably the temperature of step (b) is ambient temperature (eg around 18-22 °C) or higher (eg up to 40 °C).

Compounds of formula (I), as produced by the process of the present invention, can be used in a process for the production of compound (A) or (B) (these processes are not part of the presently claimed invention): 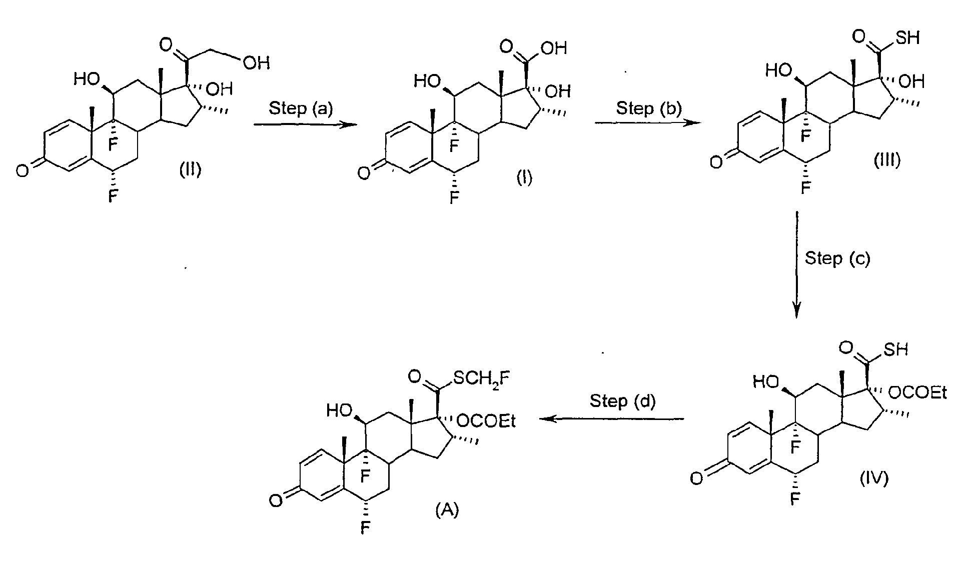 wherein step (a) comprises oxidation of a solution containing the compound of formula (II).

Step (b) will typically comprise the addition of a reagent suitable for converting a carboxylic acid to a carbothioic acid eg. using hydrogen sulphide gas together with a suitable coupling agent eg. carbonyldiimidazole (CDI) in the presence of a suitable solvent eg. dimethylformamide.
Step (c) typically comprises the addition of a reagent suitable for performing the esterification to the ethyl ester eg. propionyl chloride in the presence of suitable solvents eg. diethylamine, triethylamine, dichloromethane and acetone. Step (d) typically comprises the addition of a reagent suitable for performing alkylation eg. either by direct conversion by addition of a haloalkyl compound or via an iodinated intermediate compound.

The process for the preparation of a compound of formula (B) comprises the following steps: 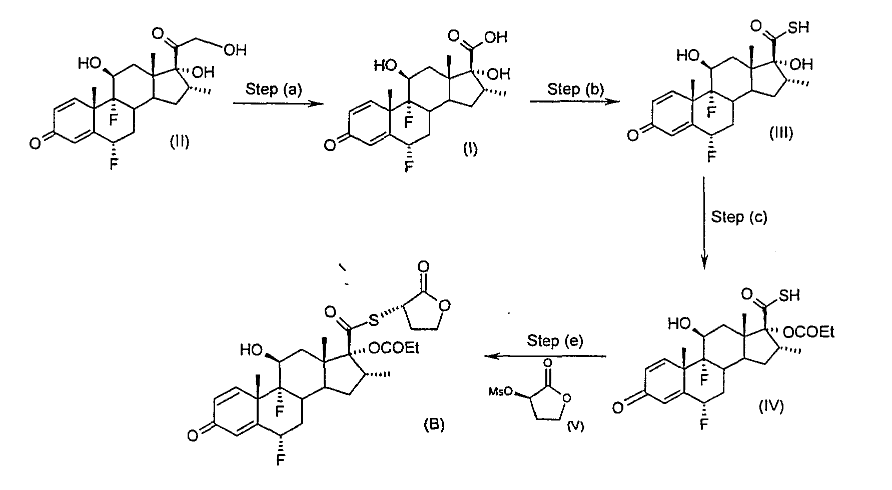 wherein step (a) comprises oxidation of a solution containing the compound of formula (II).

Typical conditions for step (b) and (c) are as previously described. Typically, step (e) will comprise reagents suitable to effect the coupling of compounds of formula (IV) with compounds of formula (V) eg. in the presence of a suitable solvent eg. dimethylformamide together with a suitable base eg. 2,4,6-collidine, pyridine or caesium carbonate. Compounds of formula (B) may then be optionally purified by suitable recrystallisation processes eg. recrystallisation from suitable solvents eg. isopropanol, diethylketone or methyl isobutyl ketone.

Compounds of formula (V) may be prepared according to the following process: 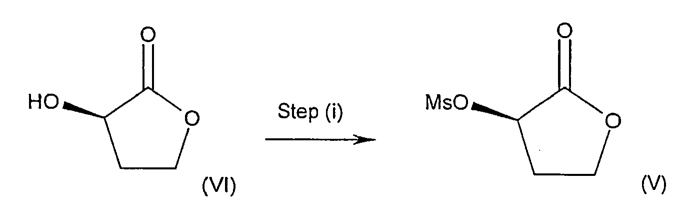 wherein step (i) typically comprises the addition of a suitable reagent eg. methanesulphonyl chloride in the presence of solvents eg. triethylamine, dimethylaminopyridine and ethyl acetate.

In step (e), analogues of compounds of formula (V) in which the MsO-group is replaced with another leaving group may also be employed.

It will be understood that this process makes use of an alternative starting material, flumethasone (formula (II)). Surprisingly, it has been shown that the use of such a starting material in the process for preparing fluticasone propionate and 6α, 9α-difluoro-11β-hydroxy-16α-methyl-3-oxo-17α-propionyloxy-androsta-1,4-diene-17β-carbothioic acid S-(2-oxo-tetrahydro-furan-3-yl) ester would substantially reduce production costs for these steroids.

The present invention is illustrated by the following Examples, wherein "Illustrative" examples are provided which illustrate the process of the claimed invention, but wherein the oxidising agent is periodic acid or sodium periodate, rather than air and/or oxygen:

### Intermediate 1: 6α, 9α-difluoro-11β, 17α-dihydroxy-16α-methyl-3-oxoandrosta-1,4-diene-17β-carbothioic acid

A solution of Example 1 (12.0g) in dry dimethylformamide (250ml) was stirred and treated with N,N'-carbonyldiimidazole (9.94g) under nitrogen at room temperature. After 4 hours, hydrogen sulphide was passed through the solution for 0.5 hours. The reaction mixture was poured into 2M hydrochloric acid (500ml) containing ice (approximately 250g). The resulting precipitate was collected, washed with water and dried in vacuo to give the title compound as a white solid (11.47g), m.p. 230-232°C, [α]_{D} +94°C (c 0.91).

### Intermediate 2: 6α, 9α-difluoro-11β-hydroxy-16α-methyl-3-oxo-17α-propionyloxy-androsta-1,4-diene-17β-carbothioic acid

A solution of Intermediate 1 (5.0g) and triethylamine (6.15ml) in dichloromethane (140ml) was cooled with ice-salt and treated dropwise with propionyl chloride (4.74ml). The reaction mixture was stirred further at approximately 0°C for 0.75 hours then washed successively with 2M sodium carbonate, water, 2M hydrochloric acid, water and brine. After being dried, solvent was removed to give a white solid (6.35g). This was redissolved in acetone (120ml) and diethylamine (12.5ml): after being stirred at room temperature for 1 hour the volume was reduced to approximately 75ml. The solution was poured into 2M hydrochloric acid (200ml) containing ice (approximately 300g) and the resulting precipitate was collected, washed with water and dried in vacuo to a white solid (5.17g) m.p. 152-155°C. Recrystallisation of a portion (400mg) from ethyl acetate gave the analytically pure title compound as colourless crystals (290mg), m.p. 161-164°C, [α]_{D} -27°C (c 0.95).

### Intermediate 3: Methansulfonic acid 2-oxo-tetrahydro-furan-3R-yl ester

Triethylamine (1.5vol, 1.1eq) and 4-N,N-dimethylaminopyridine (0.012wt, 0.01eq) in ethylacetate (2vol) is added to a stirred solution of (R)-(+)-α-hydroxy-γ-butyrolactone (1wt, 1eq) in ethyl acetate (12vol) under nitrogen at 20°C +/-3°C. The solution is cooled to below 10°C and methanesulphonyl chloride (0.79vol, 1.05eq) is cautiously added to the reaction mixture over a period of at least 15 minutes at a rate sufficient to maintain the reaction temperature below 35°C. After complete addition, the reaction mixture is cooled to 20°C +/-3°C and stirred for up to 7h at 20°C +/-3°C under nitrogen, monitoring for completion by TLC (EtOAc, cyclohexane; 1:1; staining solution: 3g KMnO₄, 20g K₂CO₃, 0.5g NaOH, and 300ml water) or GC. Upon completion, the reaction mixture is treated with 1 M HCI (3vol) and stirred until all solids have dissolved. The phases are separated and the organic phase is washed with further 1 M HCI (3vol). The phases are separated and the organic phase is distilled under reduced pressure to approximately 4vol using a rotary evaporator. The organic solution is heated to 40-50°C and treated with cyclohexane (12vol). The mixture is cooled to below 15°C and aged at 10-15°C for at least 15 minutes. The mixture is filtered, the collected solid is washed with cyclohexane (2x3vol) and dried under vacuum at 30-35°C to yield the title compound as a white solid. Expected yield: 150%w/w, 85%theory.

### Illustrative Example 1: 6α, 9α-difluoro-11β-hydroxy-16α-methyl-3-oxo-17α-hydroxy-androsta-1,4-diene-17β-carboxylic acid

A suspension of flumethasone (40g) in tetrahydrofuran (199ml) was treated with lab grade water (13.2ml) and stirred at 20°C until a clear solution was achieved (approximately 2 minutes). The solution was cooled to less than 10°C and an aqueous solution of periodic acid (99% purity, 33.32g (1.5mole equivs) in water (68ml)) was added dropwise over a period of 6 minutes. The clear solution was allowed to warm to ambient (approximately 20°C) and stirred at ambient for 2 hours and 5 minutes. HPLC analysis at 90minutes showed 97.4area% title compound present in the reaction mixture. Water (1000ml) was added dropwise over a period of 15 minutes causing crystallisation/precipitation of the product. After complete addition, the mixture was externally cooled and aged at approximately 10°C for 100minutes and the product filtered off. The bed was washed with water (3x140ml) and dried at 70°C (house vacuum) for 26hours and 40 minutes leaving the title compound as a white granular solid (37.98g, 98.3%th).

### Illustrative Example 1A: 6α, 9α-difluoro-11β-hydroxy-16α-methyl-3-oxo-17α-hydroxy-androsta-1,4-diene-17β-carboxylic acid

A suspension of flumethasone (5g, 12.2mmol) in dioxan (22ml) and lab grade water (3ml) was treated with an aqueous solution of periodic acid (50%w/w purity, 6.65g, 14.6mmol (1.2mole equivs)) over a period of 45 minutes keeping the temperature in the range 25-30°C. The suspension was stirred at ambient for 2 hours. HPLC analysis at near 2 hours showed 98.1 area% title compound present in the reaction mixture. Water (70ml) was added dropwise over a period of 45 minutes. After complete addition, the mixture was stirred 20°C for 1 hour and the product filtered off. The bed was washed with water (2x15ml) and dried at 60°C (house vacuum) for 18 hours leaving the title compound as a white granular solid (4.65g, 96.3%th).

### Illustrative Example 1B: 6α, 9α-difluoro-11β-hydroxy-16α-methyl-3-oxo-17α-hydroxy-androsta-1,4-diene-17β-carboxylic acid

A suspension of flumethasone (5g, 12.2mmol) in diethylene glygol dimethyl ether (22ml) and lab grade water (4.4ml) was treated with an aqueous solution of periodic acid (50%w/w purity, 6.65g, 14.6mmol (1.2mole equivs)) over a period of 45 minutes keeping the temperature in the range 25-30°C. The suspension was stirred at ambient for 5 hours. HPLC analysis at near 5 hours showed 95.8area% title compound present in the reaction mixture. Water (70ml) was added dropwise over a period of 45 minutes. After complete addition, the mixture was stirred at 20°C for 1 hour and the product filtered off. The bed was washed with water (2x15ml) and dried at 60°C (house vacuum) for 72 hours leaving the title compound as a white granular solid (4.66g, 96.5%th).

### Illustrative Example 1C: 6α, 9α-difluoro-11β-hydroxy-16α-methyl-3-oxo-17α-hydroxy-androsta-1,4-diene-17β-carboxylic acid

A suspension of flumethasone (5g, 12.2mmol) in tetrahydrofuran (22ml) and lab grade water (4.4ml) was treated with an aqueous solution of sodium periodate (99% purity, 3.13g, 14.6mmol (1.2mole equivs)) in hydrochloric acid (12 molar, 1.46ml 17.5mmol (1.43 equiv)) and water (8.5ml) over a period of 30 minutes keeping the temperature in the range 25-30°C. The suspension was stirred at ambient for 2 hours. HPLC analysis at near 2 hours showed 98.4area% title compound present in the reaction mixture. Water (65ml) was added dropwise over a period of 20 minutes. After complete addition, the mixture was cooled to 10°C, was stirred at 20°C for 2 hours and the product filtered off. The bed was washed with water (2x15ml) and then dried at 60°C (house vacuum) for 18 hours leaving the title compound as a white granular solid (4.65g, 96.3%th).

### Illustrative Example 1D: 6α, 9α-difluoro-11β-hydroxy-16α-methyl-3-oxo-17α-hydroxy-androsta-1,4-diene-17β-carboxylic acid

A suspension of flumethasone (1weight) in tetrahydrofuran (4.4vol) and water (0.9vol) was stirred at 22+/- 3°C until a clear solution was achieved. An aqueous solution of periodic acid (50%w/w, 1.33 weights, 1.2equivalents) was added over approximately 45 minutes at a rate sufficient to maintain a reaction temperature of 25+/-5°C. A further portion of water (0.1vol) was added as a line wash and the mixture was stirred at 22+/-3°C for 2 hours (note, the hydroxyacid product starts to crystallise after approximately 1 hour of this stir period). Water (14vol) was added to the suspension over at least 30 minutes maintaining a reaction temperature in the range 22+/-3°C. The mixture was cooled to approximately 10 °C and stirred for at least 1 hour at this temperature. The solid was filtered off and the bed washed with water (2x3vol) at 22+/-5°C. The product was dried under vacuum at 60+/-5°C to afford hydroxyacid as a white granular solid (expected yield 97%th).

### Comparative Example 2: 6α, 9α-difluoro-11β-hydroxy-16α-methyl-17α-propionyloxy-3-oxoandrosta-1, 4-diene-17β-carbothioic acid, S-fluoromethyl ester

The compound of Example 2 may be prepared from Intermediate 2 following the processes described in GB Patent No. 2288877B.

### Comparative Example 3: 6α, 9α-difluoro-11β-hydroxy-16α-methyl-3-oxo-17α-propionyloxy-androsta-1,4-diene-17β-carbothioic acid S-(2-oxo-tetrahydro-furan-3-yl) ester

A mixture of Intermediate 2 (1wt), Intermediate 3, and DMF (3.5vol) is treated with 2,4,6-collidine (0.268wt, 1.04eq). The resulting solution is heated at 39-43°C for approximately 4h until complete by HPLC. 2M Hydrochloric acid (0.2vol) is added and residual ethyl acetate is removed by vacuum distillation. The solution is warmed to 60°C and water (approximately 2vol) is added over 5-30 min at 60-65°C, seeding when a fine suspension has formed. The suspension is aged at 55-65°C for at least 5 min and water (approximately 8vol; total water added 10vol) is added slowly at 49-60°C. The suspension is allowed to cool to room temperature and is aged for at least 30min (typically overnight). The title compound is filtered off, washed with water (3 x 2vol), and pulled dry.

The title compound is then purified using isopropanol recrystallisation, which comprises heating to reflux a suspension of the title compound in isopropanol (13.4 vol) and holding at reflux for at least 5 minutes. (At this stage the reaction mixture may be given a hot filtration). The solution is heated and maintained above 60°C whilst filtered, purified water (5.6vol) is added dropwise over at least 10 minutes. The suspension is cooled to 0-10°C and then aged at <10° for at least 30 minutes. The solid is collected by vacuum filtration, washed with filtered purified water (2 x 3.4vol) and dried under vacuum using a filter bed for at least 15 minutes. The product is dried in vacuo at up to 70°C overnight. Expected yield: 99% w/w, 84% theory (uncorrected) from Intermediate 2.

The merits of the present invention may be seen by reference to the following Comparative Example:

### Comparative Example 4

Example was prepared following a procedure analogous to that described in J. Med. Chem. (1994), 37, 3717, example 2b, (half scale), which describes conversion of des-16 alpha methyl dexamethasone to corresponding hydroxyacid via a cold isolation.

A suspension of flumethasone (5.42g, 13.2mmol) in tetrahydrofuran (27.5ml) and lab grade water (3ml) was treated with an solution of periodic acid (99% purity, 4.5g, 19.8mmol (1.5mole equivs)) in lab grade water (45ml) over a period of 15 minutes keeping the temperature in the range 20-30°C. The suspension was stirred at ambient for 2 hours and an essentially clear solution resulted. HPLC analysis at near 2 hours showed 98.9area% title compound present in the reaction mixture. The reaction mixture was then added to a stirred mixture of water (75ml) and crushed ice (125g) over 5 minutes. The suspension was then stirred at 0-2°C for 10 minutes and the solid was filtered off to give a very volumous gel (*ca*100ml).

This comparative example resulted in a voluminous product with a high level of associated solvent and it was not possible to remove this solvent by conventional solvent removal techniques, such as low temperature filtration. By contrast, in Example 1A, 1B, 1C and 1D, the title compound was obtained in a relatively low volume (ca 4 ml) as a granular solid in a form which was readily filterable. In Example 1 which was performed on a larger scale the same low volume solid was obtained (ca 32 ml).

## Claims

1. A process for the preparation of a compound of formula (I) which comprises the following steps: 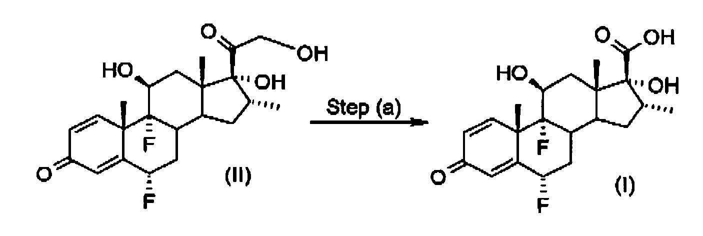
(a) oxidation of a solution containing the compound of formula (II), wherein the oxidation step comprises an oxidation reaction which utilises air and/or oxygen, followed by;
(b) precipitation of the compound of formula (I) in crystalline form from the reaction mixture by addition of an anti-solvent under temperature conditions of 10 °C or higher.

2. A process according to claim 1 wherein the solvent used in the oxidation step is methanol.

3. A process according to claim 1 or claim 2 wherein the temperature of step (b) is ambient temperature or higher.

4. A process according to any one of claims 1 to 3 wherein the anti-solvent is water.

5. A process according to any one of the preceding claims wherein step (a) is incubated at room temperature.

## Patentansprüche

1. Verfahren zur Herstellung einer Verbindung der Formel (I), das die folgenden Schritte umfaßt: 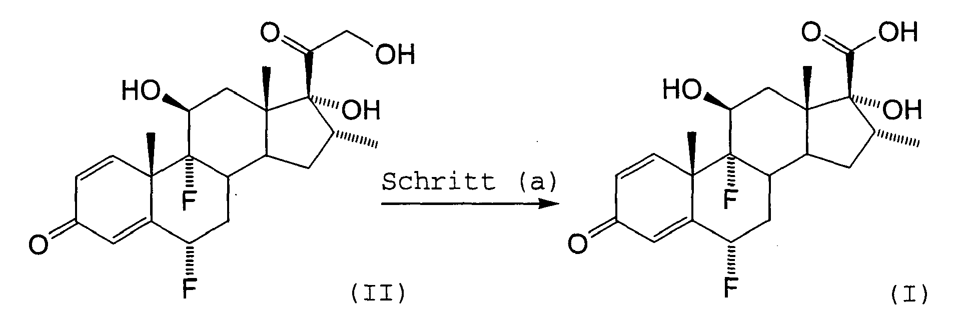
(a) Oxidation einer Lösung, die die Verbindung der Formel (II) enthält, worin der Oxidationsschritt eine Oxidationsreaktion umfaßt, die Luft und/oder Sauerstoff verwendet, gefolgt von
(b) Präzipitation der Verbindung der Formel (I) aus der Reaktionsmischung in kristalliner Form durch Zugabe eines Antilösungsmittels unter Temperaturbedingungen von 10°C oder höher.

2. Verfahren gemäß Anspruch 1, worin das Lösungsmittel, das in dem Oxidationsschritt verwendet wird, Methanol ist.

3. Verfahren gemäß Anspruch 1 oder Anspruch 2, worin die Temperatur im Schritt (b) Umgebungstemperatur oder höher ist.

4. Verfahren gemäß einem der Ansprüche 1 bis 3, worin das Antilösungsmittel Wasser ist.

5. Verfahren gemäß einem der vorhergehenden Ansprüche, worin der Schritt (a) bei Raumtemperatur inkubiert wird.

## Revendications

1. Procédé pour la préparation d'un composé de formule (I) comprenant les étapes suivantes : 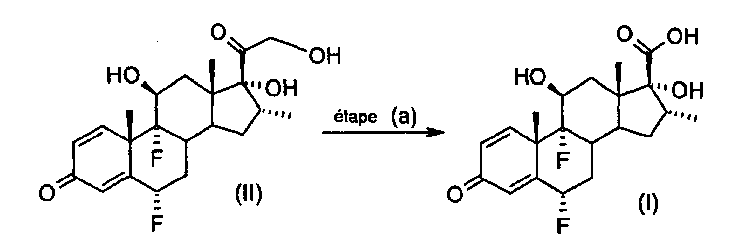
(a) oxydation d'une solution contenant le composé de formule (II), dans laquelle l'étape d'oxydation comprend une réaction d'oxydation qui utilise l'air et/ou l'oxygène, suivie par :
(b) la précipitation d'un composé de formule (I) sous sa forme cristalline à partir du mélange réactionnel par addition d'un antisolvant dans des conditions thermiques supérieures ou égales à 10°C.

2. Procédé selon la revendication 1, dans lequel le solvant utilisé dans l'étape d'oxydation est du méthanol.

3. Procédé selon la revendication 1 ou la revendication 2, dans lequel la température de l'étape (b) est supérieure ou égale à la température ambiante.

4. Procédé selon l'une quelconque des revendications 1 à 3, dans lequel l'antisolvant est de l'eau.

5. Procédé selon l'une quelconque des revendications précédentes, dans lequel l'étape (a) est incubée à température ambiante.
